(19) Europäisches Patentamt
European Patent Office
Office européen des brevets

(11) Veröffentlichungsnummer: **0 196 330 B1**

(12) **EUROPÄISCHE PATENTSCHRIFT**

(45) Veröffentlichungstag der Patentschrift: **01.07.92**

(51) Int. Cl.5: **A61K 31/40**, C12P 17/18, C07D 207/44, C07D 495/04, //(C12P17/18,C12R1:625)

(21) Anmeldenummer: **85905323.3**

(22) Anmeldetag: **19.09.85**

(86) Internationale Anmeldenummer: **PCT/EP85/00489**

(87) Internationale Veröffentlichungsnummer: **WO 86/01716 (27.03.86 86/07)**

(54) **VERWENDUNG VON PYRROTHINDERIVATEN.**

(30) Priorität: **20.09.84 DE 3434562**

(43) Veröffentlichungstag der Anmeldung: **08.10.86 Patentblatt 86/41**

(45) Bekanntmachung des Hinweises auf die Patenterteilung: **01.07.92 Patentblatt 92/27**

(84) Benannte Vertragsstaaten: **AT BE CH DE FR GB IT LI LU NL SE**

(56) Entgegenhaltungen:
**US-A- 3 014 922**

**Chemical & Pharmaceutical Bulletin, Band 28, Nr.11, November 1980 Y.T.Ninomiya et al,: "Biochemically active substances from microorganismes. V1) Pyrrothines, potent platèlet aggregation inhibitors of microbial origin", Seiten 3157-3162, siehe Seite 3157, Zusammenfassung**

(73) Patentinhaber: **BOEHRINGER MANNHEIM GMBH**
**Patentabteilung, Abt. E Sandhofer Strasse 112-132 Postfach 31 01 20**
**W-6800 Mannheim 31 Waldhof(DE)**

(72) Erfinder: **STAHL, Peter**
**Hirtenstrasse 12**
**W-8131 Bernried(DE)**
Erfinder: **SEIDEL, Hans**
**Waxensteinstrasse 6**
**W-8132 Tutzing(DE)**
Erfinder: **VON DER ELTZ, Herbert**
**Kerschensteinerstrasse 18**
**W-8120 Weilheim(DE)**
Erfinder: **WILHELMS, Otto-Henning**
**Odenwaldstrasse 25/2**
**W-6941 Weinheim-Rittenweier(DE)**
Erfinder: **ROESCH, Androniki**
**Werderstrasse 44**
**W-6800 Mannheim 1(DE)**

**EP 0 196 330 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Fortschritte der Arzneimittelforschung, Band 3, 1961, Basel (Ch) G.B. West: "A Pharmacological Approach to Allergy", Seiten 409-449, siehe Seiten 412-413; Seiten 438,439

Chemical Abstracts, Band 71, Nr. 7, 18 August 1969, Columbus, Ohio (US)W.Von Daehne et al.: "New antibiotics containing the 1,2-dithiolo (4,3-b)-pyrrole ring system", siehe Seite 168, Zusammenfassung 29128z

The Merck Index, 10. Edition, veröffentlicht 1983, Merck & Co. Inc. Rahway N.J. (US) siehe Seite 127, Zusammenfassung 885; Seite 1338, Zusammenfassung 9182

Manual of Clinical Immunology, 2, Edition, veröffentlicht 1980, American Society for Microbiology, Washington D.C. (US) R.P. Siraganian et al.: "Histamine release and assay methods for the study of human allergy" Seiten 808-821, siehe das ganze Dokument

(74) Vertreter: **Weickmann, Heinrich, Dipl.-Ing. et al**
**Patentanwälte H.Weickmann, Dr. K.Fincke F.A. Weickmann, B. Huber Dr. H. Liska, Dr. J. Prechtel Kopernikusstrasse 9 Postfach 86 08 20**
**W-8000 München 86(DE)**

## Beschreibung

Die Erfindung betrifft die Verwendung von Pyrrothinverbindungen zur Herstellung von Arzneimitteln für Hemmung der allergeninduzierten Degranulation peripherer Leukozyten.

Es ist bekannt, daß die aus Streptomyces-Kulturfiltraten erhältlichen Derivate von Pyrrothin (4-Methyl-6-amino-1,2-dithi o[4,3-b]pyrrol-5(4H)-on), wie z.B. das N-Acetyl-derivat (Thiolutin), oder das N-Propionyl-derivat (Aureothricin) Antibiotika mit Wirkung gegen grampositive und gramnegative Bakterien, pathogene Pilze und Protozoen darstellen (vgl. W.D. Celmer und I. A. Solomons, J. Am. Chem. Soc. 77 (1955) 2861); die Totalsynthese dieser Antibiotika wird von U. Schmidt und F. Geiger beschrieben (vgl. Angew. Chemie 74 (1962) 328; Liebigs Ann. Chem. 664 (1963) 168).

Es wurde nun überraschenderweise gefunden, daß solche Pyrrothinderivate auch eine ausgeprägte antiallergische Wirkung besitzen; sie hemmen z.B. die humane allergeninduzierte Degranulation peripherer Leukozyten. Eine ähnliche Wirkung zeigen auch die durch reduktive Spaltung der S-S-Bindung der Pyrrothinderivate erhältlichen entsprechenden Dimercaptoverbindungen.

Gegenstand der vorliegenden Erfindung ist deshalb die Verwendung von Pyrrothinderivaten der allgemeinen Formel I

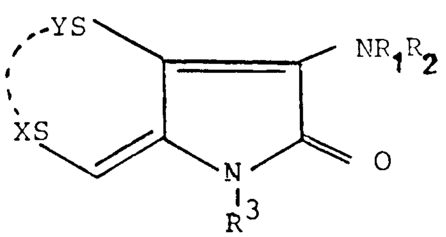

(I)

worin $R_1$ und $R_3$ Wasserstoff oder Methyl, $R_2$ Wasserstoff, Methyl oder eine Acylgruppe mit 1 bis 5, insbesondere 2 bis 4 Kohlenstoffatomen bedeuten, und X und Y Wasserstoff, ein Äquivalent eines physiologisch verträglichen Kations oder zusammen eine Einfachbindung bedeuten, zur Herstellung eines Arzneimittels für die Hemmung der allergeninduzierten Degranulation peripherer Leukozyten.

In bevorzugten Verbindungen der Formel I sind $R_1$ und $R_2$ verschieden, und insbesondere $R_1$ Wasserstoff und $R_2$ Methyl oder Acyl mit 1 bis 5, insbesondere 2 bis 4 Kohlenstoffatomen. $R_3$ ist vorzugsweise eine Methylgruppe.

Für die erfindungsgemäße Verwendung

sind die Verbindungen der Formel Ia bevorzugt, d.h. also die Verbindungen der Formel I, in denen X und Y zusammen eine Einfachbindung bilden,

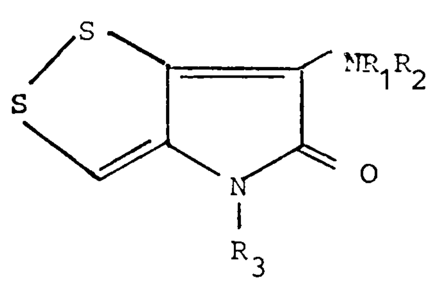

(Ia)

worin $R_1$, $R_2$ und $R_3$ die oben angegebene Bedeutung besitzen.

Die Verbindungen der Formel I, in denen X und Y zusammen keine Einfachbindung bilden, sondern ein Wasserstoffatom oder ein Äquivalent eines physiologisch verträglichen Kations bedeuten, werden durch die Formel Ib dargestellt:

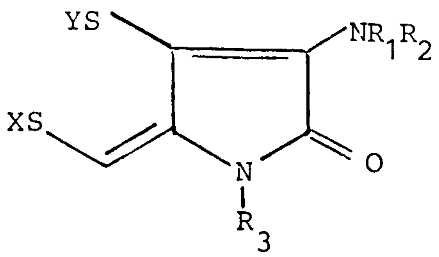

(Ib)

worin $R_1$, $R_2$ und $R_3$ die oben angegebenen Bedeutungen besitzen. Sie liegen vorzugsweise in Form ihrer Salze (X, Y = Äquivalent eines physiologisch verträglichen Kations) vor.

Die Verbindungen der Formel I, worin $R_1$, $R_2$ und $R_3$ Methyl und X und Y Wasserstoff, ein Äquivalent eines physiologisch verträglichen Kations oder zusammen eine Einfachbindung bedeuten, sind neue Verbindungen.

Die Verbindungen der allgemeinen Formel Ib zeigen bei der erfindungsgemäßen Verwendung die gleiche antiallergische Wirksamkeit wie die Verbindungen der Formel Ia.

Eine Acylgruppe mit 1 bis 5 Kohlenstoffatomen kann geradkettig oder verzweigt sein und ist z. B. Valeryl, Isovaleryl und Trimethylacetyl, und insbesondere Butyryl, Isobutyryl, Propionyl, Acetyl und Formyl.

Ein physiologisch verträgliches Kation X und Y kann ein- oder mehrwertig sein und ist insbesondere ein ein- oder zweiwertiges physiologisch verträgliches Kation, wie z.B. $Ca^{2+}$, $Na^{+}$ oder $K^{+}$; es kann aber auch Ammonium oder ein organisches Ammonium-Kation, wie z.B. Mono-, Di- oder Trialkylammonium sein, wobei die Alkylgruppe dann insbesondere Methyl bedeutet.

Die Verbindungen der Formel Ia sind bekannt oder lassen sich auf an sich bekannte Weise auf synthetischem Wege oder aus Actinomycetales, insbesondere Streptomyces-Kulturfiltraten, herstellen (vgl. The Chemistry of Heterocyclic Compounds, Part 1, D.S. Breslow, H. Skolnik, Interscience Publishers, 1966, Seite 420; U. Schmidt und F. Geiger, Angew. Chemie 74 (1962), 328; Liebigs Ann. Chem. 664 (1963), 168).

Die Verbindungen der Formel Ib, in denen X und Y ein Wasserstoffatom bedeuten, lassen sich aus den entsprechenden Verbindungen der Formel Ia auf an sich bekannte Weise durch reduktive Ringspaltung mit komplexen Hydriden, insbesondere mit Natriumborhydrid, erhalten (vgl. U. Schmidt und F. Geiger, Liebigs Ann. Chem. 664 (1963), 168); diese Verbindungen lassen sich auf übliche Weise in ihre Salze überführen, in denen X und Y ein pharmazeutisch verträgliches Kation darstellen, und werden vorzugsweise in Form ihrer Salze verwendet, z. B. direkt in der durch reduktive Spaltung von Ia erhaltenen Form bzw. Lösung.

Erfindungsgemäß können die Verbindungen der Formel I einzeln oder als Gemisch zwei oder mehrerer Verbindungen verwendet werden. Es kann deshalb auch das aus bestimmten Actinomycetales-Arten gewonnene Wirkstoffgemisch (vgl. z.B. W. D. Celmer, I.A. Solomons, J. Am. Chem. Soc. 77 (1955) 2861; U. Schmidt und F. Geiger, Angew. Chemie 74 (1962) 328) eingesetzt werden. Es wurde gefunden, daß sich ein vom Gesichtspunkt der Herstellung (Wirkstoffausbeute) und der erfindungsgemäßen Verwendung gut geeignetes Wirkstoffgemisch zweckmäßigerweise aus Streptoverticillium thioluteum (DSM Nr. 40027) und insbesondere aus seiner Kulturbrühe (Kulturfiltrat) erhalten läßt. Die Aufarbeitung kann dabei auf für derartige Verfahren an sich bekannte Weise erfolgen, wie z. B. Einengen und/oder Extraktion der Kulturbrühe. Als hauptsächliche Wirkstoffkomponenten des Kulturfiltrats von Streptoverticillium thioluteum wurden festgestellt: ca. 70 % Aureothricin (MG = 242, Formel Ia: $R_1$ = H, $R_2$ = Propionyl, $R_3$ = $CH_3$); ca. 15 % Thiolutin (MG = 228, Formel Ia: $R_1$ = H, $R_2$ = Acetyl, $R_3$ = $CH_3$); ca. 10 % Isobutyrylpyrrothin (MG = 256; Formel Ia: $R_1$ = H, $R_2$ = Isobutyryl, $R_3$ = $CH_3$).

Für den Nachweis der antiallergischen Wirkung wurde die Hemmung der allergeninduzierten Degranulationsreaktion peripherer Leukozyten gemessen; es wurde nach der Methode von R.P. Siraganian und W.A. Hook (Manual of Clinical Immunology, 2. Auflage 1980, Herausg. N.R. Rose und H. Friedman, Am. Soc. for Microbiology, Seiten 808 bis 821) gearbeitet. Als Vergleichssubstanz wurde der bekannte Degranulationsinhibitor Theophyllin herangezogen.

In der nachfolgenden Tabelle I sind die Hemmdaten $ID_{50}$ (mol/l für 50%ige Hemmung in vitro) und Toxizitäten $LD_{50}$ (p.o., mg/kg Maus; zur Toxizität vgl. auch 118. Am. Chem. Soc. Meet., Chicago, Sept. 1950, Abstracts of Papers, Seite 18a; Seneca et al, Antibiol. + Chemother. 2, 357 (1952); US-Patent 2 798 811) von erfindungsgemäß verwendeten Verbindungen und die der Vergleichssubstanz Theophyllin angegeben.

Tabelle I

| Verbindung | $ID_{50}$ | $LD_{50}$ |
|---|---|---|
| Pyrrothin | $9 \times 10^{-7}$ | - |
| Thiolutin | $4 \times 10^{-7}$ | 25 |
| Aureothricin | $9 \times 10^{-7}$ | - |
| Isobutyrylpyrrothin | $1{,}7 \times 10^{-6}$ | - |
| Ib, $R_1 = H$, $R_2 = COCH_3$, $R_3 = CH_3$, als Na-Salz | $2{,}5 \times 10^{-6}$ | - |
| Ia, $R_1, R_2, R_3 = CH_3$ | $4{,}6 \times 10^{-6}$ | - |
| Ib, $R_1, R_2, R_3 = CH_3$, als Na-Salz | $5{,}0 \times 10^{-6}$ | - |
| Theophyllin | $5 \times 10^{-3}$ | 540 |

Aus den Werten der Tabelle I errechnet sich für Thiolutin ein therapeutischer Index $LD_{50}/ID_{50}$ von $6{,}2 \times 10^7$; für die Vergleichsverbindung Theophyllin ein solchern von $0{,}8 \times 10^3$; die erfindungsgemäß eingesetzte Verbindung besitzt also einen wesentlich höheren Faktor (um mehr als $10^4$!), der die größere pharmazeutische Breite und die damit verbundene gute Eignung der erfindungsgemäß verwendeten Verbindungen veranschaulicht.

Die erfindungsgemäß verwendeten Verbindungen der allgemeinen Formel I können zur Herstellung der üblichen, insbesondere für Antiallergika geeigneten pharmazeutischen Zubereitungen und Darreichungsformen verwendet werden, z. B. als Tabletten, Dragees, Suppositorien, Injektionslösungen, Säfte, Inhalationssprays usw. Die Arzneimittel enthalten den Wirkstoff vorzugsweise zusammen mit üblichen pharmazeutischen Träger- und Verdünnungsmitteln, gegebenenfalls auch in Kombination mit anderen Wirkstoffen, wie z.B. weiteren Antiallergika, oder für die Allergietherapie weiteren geeigneten Wirkstoffen, wie z.B. fiebersenkenden Mitteln, antiinflammatorisch wirkenden Mitteln, Vitaminen, usw. Die tägliche Verabreichungsdosis richtet sich insbesondere nach der Art und Schwere der Erkrankung; sie beträgt beim erwachsenen Menschen in der Regel 0,1 bis 10 mg Wirksubstanz.

**Beispiele**

Beispiel 1:

Anzucht von Streptoverticillium thioluteum DSM 40027

| Zusammensetzung des Zuchtmediums: | |
|---|---|
| Stärke (separat erhitzt) | 20 g |
| Pepton aus Fleisch, tryptisch verdaut (Merck) | 5 g |
| Hefeextrakt (Difco) | 4 g |
| $CaCO_3$, gefällt | 2 g |
| $KNO_3$ | 1 g |
| $K_2HPO_4$ | 0,5 g |
| $MgSO_4 \cdot 7H_2O$ | 1 g |
| NaCl | 0,5 g |
| $FeSO_4 \times 7H_2O$ | 0,02 g |
| gelöst in 1 l Leitungswasser, pH ca. 7,0. | |

In diesem Medium werden unter Standardfermentationsbedingungen (Fermentationsdauer 24 bis 56 Stunden) bei gutem Wachstum hohe Syntheseleistungen erzielt.

Zur Isolierung eines Wirkstoffgemisches wird das Kulturfiltrat durch Zentrifugation gewonnen.

**Beispiel 2**

Isolierung reiner Wirkstoffe aus Streptoverticillium thioluteum DSM 40027.

Das nach Beispiel 1 erhaltene Kulturfiltrat (20 l, pH = 6) wurde auf 2 l eingeengt und mit Essigester extrahiert. Die organische Phase wurde eingeengt, und gegebenenfalls mit Kieselgel/Chloroform chromatographiert; als Rückstand verbleibt ein stark gelb gefärbtes Pulver; nach Umkristallisieren aus Aceton werden heterogene Kristalle (ca. 0,4 g) erhalten. Zur Auftrennung des Gemisches werden die Kristalle einer weiteren Chromatographie an Kieselgel (Methylenchlorid → Methylenchlorid/Aceton = 8/2) unterworfen. Es wurden die Fraktionen 1 (ca. 35 mg), 2 (ca. 165 mg), die Mischfraktionen 1/2 (ca. 45 mg) und die Fraktion 3 (ca. 55 mg) eluiert. Die Fraktionen entsprechen den folgenden bekannten Verbindungen: 1 = Isobutyrylpyrrothin, 2 = Aureothricin, 3 = Thiolutin.

**Beispiel 3**

3-Acetamido-4-mercapto-5-mercaptomethylen-1-methyl-pyrrolin-2-on-dinatriumsalz der Strukturformel

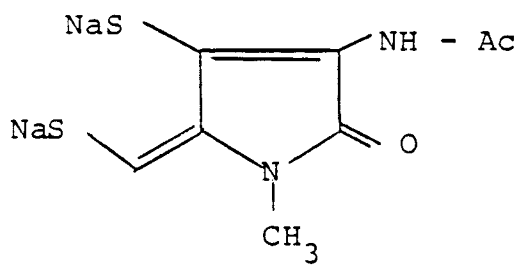

Thiolutin-Reduktion:
228 mg (1 mmol) Thiolutin werden in 100 ml Wasser suspendiert und mit 76 mg (2 mmol) $NaBH_4$ versetzt. Man rührt bei Raumtemperatur, bis eine klare Lösung entstanden ist. Das Produkt kann unter Stickstoffatmosphäre chromatographisch (Kieselgel) aufgereinigt werden.
$R_F$ = 0,58, $CHCl_3/CH_3OH$ 1/1
$ID_{50} = 2{,}5 \times 10^{-6}$

**Beispiel 4**

N,N-Dimethyl-pyrrothin der Strukturformel

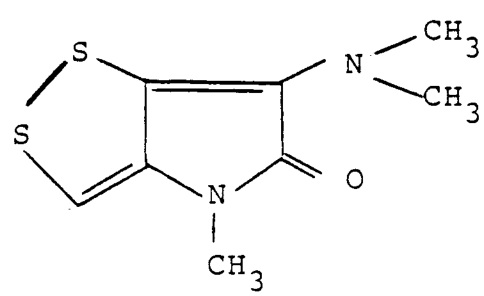

1,14 g (5 mmol) Thiolutin werden entsprechend der Vorschrift von CELMER und SOLOMONS J. Am. Chem. Soc. 77 (1955) 2861 zum Pyrrothin-Hydrochlorid verseift, und mit überschüssigem Ammoniak in die freie Base überführt. Diese wird in 10 ml Acetonitril und 5 ml Formalin aufgenommen und mit 0,95 g $NaCNBH_3$ versetzt. Unter Rühren tropft man während 30 Minuten 0,5 ml Eisessig zu, rührt 2 Stunden bei Raumtemperatur und fügt nochmals 0,5 ml Eisessig zu. Nach weiteren 2 Stunden rühren verdünnt man mit Ether und extrahiert mit 1 N KOH sowie mit Wasser. Die organische Phase wird getrocknet und im Vakuum zur Trockne eingedampft. Der Rückstand wird über Kieselgel (Fließmittel: $CHCl_3$) chromatographiert.
$R_F$ = 0,66, $CHCl_3$/Aceton 9/1
$ID_{50} = 4{,}6 \times 10^{-6}$

**Beispiel 5**

3-Dimethylamino-4-mercapto-5-mercaptomethylen-1-methyl-pyrrolin-2-on-dinatriumsalz der Strukturformel

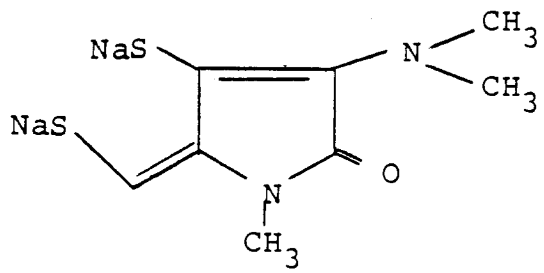

N,N-Dimethyl-pyrrothin-Reduktion:
214 mg (1mmol) N,N-Dimethyl-pyrrothin werden in 100 ml Wasser suspendiert und mit 76 mg (2 mmol) $NaBH_4$ versetzt. Man rührt, bis eine klare Lösung entstanden ist. Das Produkt kann unter Stickstoffatmosphäre chromatographisch (Kieselgel) aufgereinigt werden.
$R_F$ = 0,65, $CHCl_3/CH_3OH$ 1/1
$ID_{50}$ = 5 x $10^{-5}$

**Patentansprüche**

**Patentansprüche für folgende Vertragsstaaten : BE, CH, DE, FR, GB, IT, LI, LU, NL, SE**

**1.** Verwendung von Pyrrothinderivaten der allgemeinen Formel I

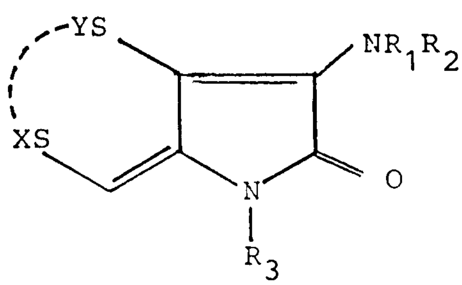

(I)

worin $R_1$ und $R_3$ Wasserstoff oder Methyl, $R_2$ Wasserstoff, Methyl oder eine Acylgruppe mit 1 bis 5 Kohlenstoffatomen bedeuten, und X und Y Wasserstoff, ein Äquivalent eines physiologisch verträglichen Kations oder zusammen eine Einfachbindung bedeuten, zur Herstellung eines Arzneimittels für die Hemmung der allergeninduzierten Degranulation peripherer Leukozyten.

**2.** Verwendung nach Anspruch 1, dadurch gekennzeichnet, daß in den Verbindungen der Formel I $R_1$ Wasserstoff, $R_2$ Methyl oder Acyl mit 1 bis 5, insbesondere 2 bis 4 Kohlenstoffatomen, und $R_3$ Methyl bedeuten.

**3.** Verwendung nach Anspruch 1 oder 2, dadurch gekennzeichnet, daß man die Verbindungen der Formel Ia

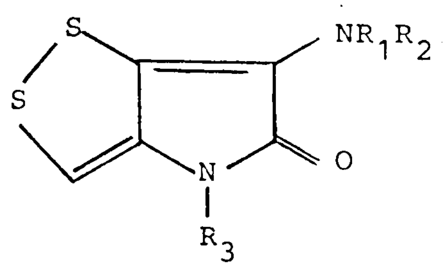

(Ia)

einsetzt, worin $R_1$, $R_2$ und $R_3$ die in Anspruch 1 oder 2 angegebene Bedeutung besitzen.

**4.** Verwendung des aus Streptoverticillium thioluteum (DSM-Nr. 40027) oder seiner Kulturbrühe gewonnenen Pyrrothinderivatgemisches für den Zweck von Anspruch 1.

**5.** Pyrrothinderivate der allgemeinen Formel I nach Anspruch 1, worin $R_1$, $R_2$ und $R_3$ Methyl und X und Y Wasserstoff, ein Äquivalent eines physiologisch verträglichen Kations oder zusammen eine Einfachbindung bedeuten.

**Patentansprüche für folgenden Vertragsstaat : AT**

**1.** Verwendung von Pyrrothinderivaten der allgemeinen Formel I

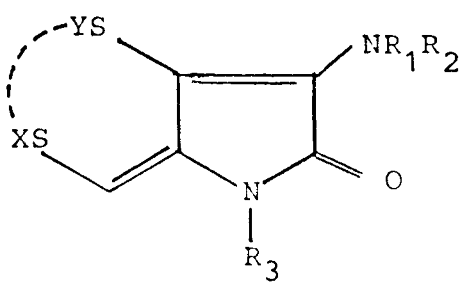

(I)

worin $R_1$ und $R_3$ Wasserstoff oder Methyl, $R_2$ Wasserstoff, Methyl oder eine Acylgruppe mit 1 bis 5 Kohlenstoffatomen bedeuten, und X und Y Wasserstoff, ein Äquivalent eines physiologisch verträglichen Kations oder zusammen eine Einfachbindung bedeuten, zur Herstellung eines Arzneimittels für die Hemmung der allergeninduzierten Degranulation peripherer Leukozyten.

**2.** Verwendung nach Anspruch 1, dadurch gekennzeichnet, daß in den Verbindungen der Formel I $R_1$ Wasserstoff, $R_2$ Methyl oder Acyl mit 1 bis 5, insbesondere 2 bis 4 Kohlenstoffatomen, und $R_3$ Methyl bedeuten.

**3.** Verwendung nach Anspruch 1 oder 2, dadurch gekennzeichnet, daß man die Verbindungen der Formel Ia

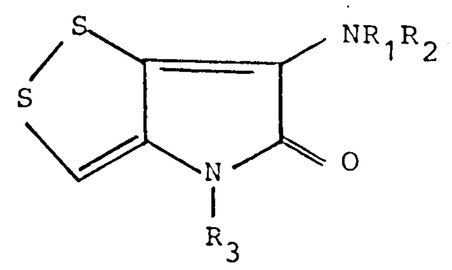

(Ia)

einsetzt, worin $R_1$, $R_2$ und $R_3$ die in Anspruch 1 oder 2 angegebene Bedeutung besitzen.

4. Verwendung des aus Streptoverticillium thioluteum (DSM-Nr. 40027) oder seiner Kulturbrühe gewonnenen Pyrrothinderivatgemisches für den Zweck von Anspruch 1.

**Claims**

**Claims for the following Contracting States : BE, CH, DE, FR, GB, IT, LI, LU, NL, SE**

1. Use of pyrrothine derivatives of the general formula I

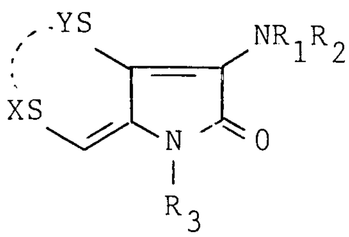

(I)

wherein $R_1$ and $R_3$ signify hydrogen or methyl, $R_2$ hydrogen, methyl or an acyl group with 1 to 5 carbon atoms and X and Y hydrogen, an equivalent of a physiologically acceptable cation or together a single bond, for the preparation of a medicament for the inhibition of the allergen-induced degranulation of peripheral leukocytes.

2. Use according to claim 1, characterised in that, in the compounds of the formula I, $R_1$ signifies hydrogen, $R_2$ methyl or acyl with 1 to 5, especially 2 to 4 carbon atoms and $R_3$ methyl.

3. Use according to claim 1 or 2, characterised in that one uses the compounds of the formula Ia

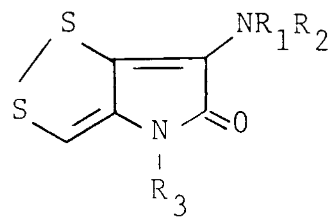

(Ia)

wherein $R_1$, $R_2$ and $R_3$ possess the meaning given in claim 1 or 2.

4. Use of the pyrrothine derivative mixture obtained from Streptoverticillium thioluteum (DSM No. 40027) or its culture broth for the purpose of claim 1.

5. Pyrrothine derivatives of the general formula I according to claim 1, wherein $R_1$, $R_2$ and $R_3$ signify methyl and X and Y hydrogen, an equivalent of a physiologically acceptable cation or together a single

bond.

**Claims for the following Contracting State : AT**

**1.** Use of pyrrothine derivatives of the general formula I

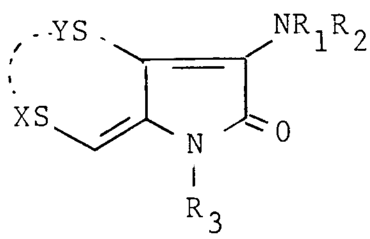

(I)

wherein $R_1$ and $R_3$ signify hydrogen or methyl, $R_2$ hydrogen, methyl or an acyl group with 1 to 5 carbon atoms and X and Y hydrogen, an equivalent of a physiologically acceptable cation or together a single bond, for the preparation of a medicament for the inhibition of the allergen-induced degranulation of peripheral leukocytes.

**2.** Use according to claim 1, characterised in that, in the compounds of the formula I, $R_1$ signifies hydrogen, $R_2$ methyl or acyl with 1 to 5, especially 2 to 4 carbon atoms and $R_3$ methyl.

**3.** Use according to claim 1 or 2, characterised in that one uses the compounds of the formula Ia

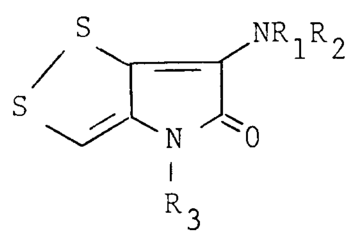

(Ia)

wherein $R_1$, $R_2$ and $R_3$ possess the meaning given in claim 1 or 2.

**4.** Use of the pyrrothine derivative mixture obtained from Streptoverticillium thioluteum (DSM No. 40027) or its culture broth for the purpose of claim 1.

**Revendications**

**Revendications pour les Etats contractants suivants : BE, CH, DE, FR, GB, IT, LI, LU, NL, SE**

**1.** Utilisation de dérivés de la pyrrothine de formule générale I

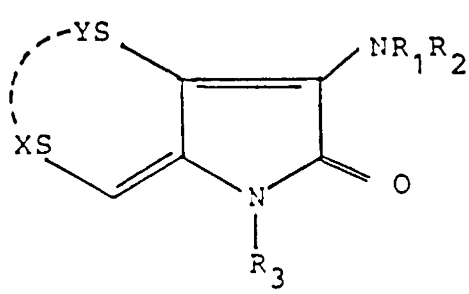

(I)

dans laquelle $R_1$ et $R_3$ représentent l'hydrogène ou un groupe méthyle, $R_2$ représente l'hydrogène, un groupe méthyle ou un groupe acyle de 1 à 5 atomes de carbone, et X et Y représentent l'hydrogène,

un équivalent d'un cation acceptable du point de vue physiologique ou représentent ensemble une liaison simple, pour la préparation d'un médicament pour l'inhibition de la dégranulation des leucocytes périphériques induite par des allergènes.

2. Utilisation selon la revendication 1, caractérisée en ce que, dans les composés de formule I, $R_1$ représente l'hydrogène, $R_2$ représente un groupe méthyle ou un groupe acyle de 1 à 5, en particulier de 2 à 4 atomes de carbone, et $R_3$ représente un groupe méthyle.

3. Utilisation selon la revendication 1 ou 2, caractérisée en ce que l'on utilise les composés de formule Ia

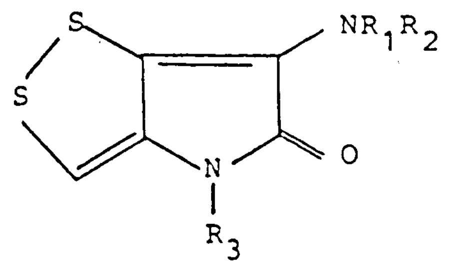

(Ia)

dans laquelle $R_1$, $R_2$ et $R_3$ possèdent la signification indiquée dans la revendication 1 ou 2.

4. Utilisation du mélange de dérivés de la pyrrothine obtenu à partir de Streptoverticillium thioluteum (DSM N°. 40027) ou de son bouillon de culture pour l'objet de la revendication 1.

5. Dérivés de la pyrrothine de formule générale I selon la revendication 1, dans laquelle $R_1$, $R_2$ et $R_3$ représentent un groupe méthyle et X et Y représentent l'hydrogène, un équivalent d'un cation acceptable du point de vue physiologique ou représentent ensemble une liaison simple.

**Revendications pour l'Etat contractant suivant : AT**

1. Utilisation de dérivés de la pyrrothine de formule générale I

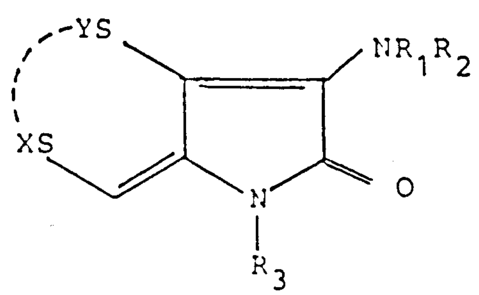

(I)

dans laquelle $R_1$ et $R_3$ représentent l'hydrogène ou un groupe méthyle, $R_2$ représente l'hydrogène, un groupe méthyle ou un groupe acyle de 1 à 5 atomes de carbone, et X et Y représentent l'hydrogène, un équivalent d'un cation acceptable du point de vue physiologique ou représentent ensemble une liaison simple, pour la préparation d'un médicament pour l'inhibition de la dégranulation des leucocytes périphériques induite par des allergènes.

2. Utilisation selon la revendication 1, caractérisée en ce que, dans les composés de formule I, $R_1$ représente l'hydrogène, $R_2$ représente un groupe méthyle ou un groupe acyle de 1 à 5, en particulier de 2 à 4 atomes de carbone, et $R_3$ représente un groupe méthyle.

3. Utilisation selon la revendication 1 ou 2, caractérisée en ce que l'on utilise les composés de formule Ia

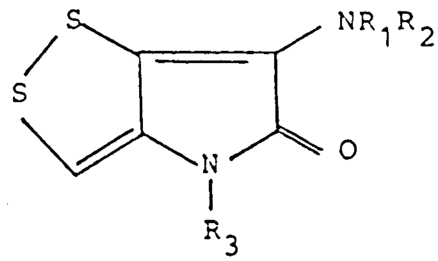

(Ia)

dans laquelle $R_1$, $R_2$ et $R_3$ possèdent la signification indiquée dans la revendication 1 ou 2.

**4.** Utilisation du mélange de dérivés de la pyrrothine obtenu à partir de Streptoverticillium thioluteum (DSM N°. 40027) ou de son bouillon de culture pour l'objet de la revendication 1.